# EUROPEAN PATENT APPLICATION

(11) **EP 3 741 331 A1**
(43) Date of publication of application: **25.11.2020**
(21) Application number: 19176333.3
(22) Date of filing: 24.05.2019
(51) Int. Cl.: A61F 2/90, A61B 17/11, A61F 2/06, A61F 2/88

(54) **STENT FOR CONNECTING DISSIMILAR ORGANS WITH PIGTAIL STRUCTURE**

(71) Applicant: Seoul National University Hospital, Seoul 03080 (KR); S&G Biotech, Inc., Yongin-si, Gyeonggi-do 17023 (KR)
(72) Inventor: KANG, Sung Kwon, 16876 Gyeonggi-do (KR); LEE, Sang Hyub, 06276 Seoul (KR); LEE, Young Jae, 13428 Gyeonggi-do (KR)
(74) Representative: Zumstein, Angela

(57) **Abstract**

The present invention relates to a stent with a pigtail structure used for connection between organs of different kinds where a through hole is formed on each of the organs of different kinds and portions around the through holes are connected to each other, the stent comprising: a stent body where both end parts are arranged across the through holes formed on the organs of different kinds and a connecting passage which connects the two organs of different kinds is formed; and a one-side wound end part and an other-side wound end part which are formed in a pigtail shape at both end parts of the stent body to be caught and fixed to the through holes such that portions of the organs of different kinds around the through holes are in close contact with each other, wherein the stent body, the one-side wound end part, and the other-side wound end part are provided as an integral body having a mesh structure made of woven wire and comprising a cover film which covers the woven wire for sealing of the connecting passage.

The present invention has the effects of facilitation of securing and maintaining a sufficient passage diameter for movement of surgical instruments, being free of the risk of deviating from a position where it is installed by securing sufficient deviation prevention, and, due to a variable shape of wire crossing of the stent connecting organs of different kinds, minimization of metal fatigue of the wire caused in the process of securing and maintaining a passage diameter for movement of surgical instruments such as an endoscope against external forces exerted on the stent to consequently lengthens lifetime of the wire constituting the stent.

## Description

### [TECHNICAL FIELD]

The present invention relates to a stent for connection between organs of different kinds with a pigtail structure and, more specifically to a stent for connection between organs of different kinds with a pigtail structure that facilitates securing and maintaining a sufficient passage diameter for movement of surgical instruments and has no risk of deviation from a position where it is installed by achieving sufficient deviation prevention.

### [BACKGROUND ART]

Typically, ingested food goes through the esophagus and is temporarily stored in the stomach. Then, it passes through the digestive organs including the small intestine divided into the duodenum, jejunum, and ileum and the large intestine sequentially.

Meanwhile, in a case where some digestive organs do not function normally or need to be removed due to development of a lesion such as a malignant tumor, a surgery should be performed to remove the portion and connect the separated digestive organs. For example, in a case where the food stored in the stomach needs to bypass the duodenum or the duodenum needs to be removed due to a serious disease in the duodenum such as an inflammation or a malignant tumor, it is necessary to connect the stomach directly to the jejunum.

Conventionally, organs of different kinds were connected through a surgical operation where a hole is created in each of the stomach and the jejunum and the portions around the holes are stitched.

However, such a surgery had a problem that it is time-consuming and requires a long period for recovery after the surgery.

In this regard, a stent for anastomosis was provided that was capable of connecting organs of different kinds using an endoscope without a surgical operation.

In general, a stent is used to expand a portion narrowed by stenosis and is configured according to its use based on the size, properties and environment of the organ or lumen it is installed at so as to solve the blockage or inhibition of the flow of substances caused due to stenosis of the lumen and to maintain a sufficient diameter of the lumen for a long period of time.

However, in addition to its use for the expansion of a narrowed portion, a variety of forms of use of stents are presented recently; they are also used for drainage of a pseudocyst due to pancreatitis where the stomach and the pseudocyst of the pancreas are connected by a stent through an endoscopic procedure to discharge the pseudocyst and relieve symptoms. In addition, they can also be used to drain liver abscess to the stomach or, used as a bypass to discharge bile by connecting the gallbladder to the stomach or the duodenum, or for drainage from the biliary tract to the stomach. They are used to guide a movement path of various surgical instruments and create an additional movement path of surgical instruments, as well as to recover the diameter of a movement path of substances by expansion.

A prior document, Korean Patent Laid-Open No. 10-2013-0110413, relating to conventional art of using stents in the forms other than expansion of a portion narrowed by stenosis discloses "a stent for anastomosis where through holes are formed on organs of different kinds and the portions around the holes are anastomosed, the stent comprising: a body part where both end parts are arranged across the through holes formed on the organs of different kinds and a connecting passage which connects the organs of different kinds is provided; flare parts which diverge to outside at both end parts of the body part and are inclined from the center of the body part toward the end parts to face the inner surfaces of the organs around the through holes to keep a close contact; and

flap parts which protrude from the both end parts of the body part such that portions of the organs of different kinds around the through holes arranged between the flare parts come into close with each other and which is inclined from the end parts of the body part to the center to maintain the state of supporting the inner sides of the organs around the through holes, whereby the state is maintained where the portions around the through holes formed on the organs of different kinds are in contact with each other, as a result of which the organs of different kinds are connected, wherein the body part, the flare parts, and the flap parts are provided as an integral body having a mesh structure of woven wire, and comprising a cover film which covers the woven wire for sealing of the connecting passage" in which a separate connecting passage that connects organs of different kinds is formed to achieve and maintain the connection of the organs of different kinds.

However, in such conventional art, organs of different kinds come into close contact to maintain the state of being joined for the purpose of anastomosis and the flare parts extending from both end parts of the body are formed at an obtuse angle, which has problems of difficulty in securing and maintaining a sufficient passage diameter for movement of surgical instruments and of deviation from the position where it is installed due to difficulty in securing sufficient deviation prevention.

Additionally, since the wire crisscrossing form of the stent connecting organs of different kinds is uniformly made, which leads to a problem of increase in metal fatigue of the wire caused in the process of securing and maintaining a passage diameter for movement of surgical instruments against external forces exerted on the stent, which consequently shortens the lifetime of the wire constituting the stent.

### [PRIOR ART DOCUMENT]

### [PATENT DOCUMENT]

(Patent Document 0001) Korean Patent Laid-Open No. 10-2013-0110413

### [CONTENTS OF THE INVENTION]

### [PROBLEM TO BE SOLVED]

The present invention, which is intended to solve the problems mentioned above, has objects of providing a stent for connection between organs of different kinds with a pigtail structure that has no risk of deviating from a position where it is installed by securing sufficient deviation prevention and, due to a variable shape of wire crisscrossing form of the stent connecting organs of different kinds, minimizes metal fatigue of the wire caused in the process of securing and maintaining a passage diameter for movement of surgical instruments such as an endoscope against external forces exerted on the stent to consequently lengthen the lifetime of the wire constituting the stent.

The objects that prevent invention seeks to achieve are not limited to the above-mentioned objects, and other objects not mentioned could be obviously understood by a skilled person in the art in view of the following descriptions.

### [MEANS FOR SOLVING THE PROBLEM]

In order to achieve the objects, the present invention provides a stent with a pigtail structure for connection between organs of different kinds where a through hole is formed on each of the organs of different kinds and portions around the through holes are connected to each other, the stent comprising:

a stent body where both end parts are arranged across the through holes formed on the organs of different kinds and a connecting passage which connects the two organs of different kinds is formed; and

a one-side wound end part and an other-side wound end part which are formed in a pigtail shape at both end parts of the stent body to be caught and fixed to the through holes such that portions of the organs of different kinds around the through holes are in close contact with each other,

characterized in that the stent body, the one-side wound end part, and the other-side wound end part are provided as an integral body having a mesh structure made of woven wire, and that it is further provided with a cover film which covers the woven wire for sealing of the connecting passage.

The wound end part is characterized in that it is a one-side wound end part formed only on one side of the end parts of the stent body.

The wound end part is characterized in that it is a one-side wound end part and an other-side wound end part formed on both sides of the end parts of the stent body.

The one-side wound end part is characterized in that from the stent body, it is wound 360 degrees from the top right toward the bottom right and the other-side wound end part is characterized in that from the stent body, it is wound 360 degrees from the bottom left toward the top left.

The one-side wound end part is characterized in that from the stent body, it is wound 360 degrees from the top right toward the bottom right and the other-side wound end part is characterized in that from the stent body, it is wound 360 degrees from the bottom right toward the top right.

The one-side wound end part is characterized in that from the stent body, it is wound 360 degrees from the top left toward the bottom left and the other-side wound end part is characterized in that from the stent body, it is wound 360 degrees from the bottom left toward the top left.

The one-side wound end part is characterized in that from the stent body, it is wound 360 degrees from the top left toward the bottom left and the other-side wound end part is characterized in that from the stent body, it is wound 360 degrees from the bottom right toward the top right.

The one-side wound end part is characterized in that from the stent body, it is wound 360 degrees from the top right toward the bottom right and the other-side wound end part is characterized in that from the stent body, it is wound 360 degrees from the bottom left toward the top left.

The stent body is characterized in that it has a length of 20-40mm and a diameter of 6-12mm, the total length comprising the stent body, the one-side wound end part, and the other-side wound end part being 130-150mm and the cover film having a thickness of 4-8mm.

### [EFFECT OF THE INVENTION]

As can be seen above, the present invention can facilitate securing and maintaining a sufficient passage diameter for movement of surgical instruments, be free of the risk of deviating from a position where it is installed by securing sufficient deviation prevention, and, due to a variable shape of the wire crisscrossing form of the stent connecting organs of different kinds, minimize metal fatigue of the wire caused in the process of securing and maintaining a passage diameter for movement of surgical instruments such as an endoscope against external forces exerted on the stent to consequently lengthen the lifetime of the wire constituting the stent.

The present invention is not limited to the above-mentioned specific preferred examples. A person having common knowledge in the technical field to which the present invention pertains will obviously be able to perform various modifications thereof without deviating from the spirit and scope of the invention as defined in the claims, and such modifications will be within the scope of the claims.

### [BRIEF DESCRIPTION OF THE DRAWINGS]

FIG. 1 is a front view of an example of the stent for connection between organs of different kinds with a pigtail structure according to the present invention.)
FIG. 2 is a top view of an example of the stent for connection between organs of different kinds with a pigtail structure according to the present invention.
FIG. 3 is a right view of an example of the stent for connection between organs of different kinds with a pigtail structure according to the present invention.
FIG. 4 is a bottom view of an example of the stent for connection between organs of different kinds with a pigtail structure according to the present invention.
FIG. 5 is a front view of an example of the stent for connection between organs of different kinds with a pigtail structure according to the present invention during operation.

### [DETAILED DESCRITPION TO CARRY OUT THE INVENTION]

The present invention is hereinafter described in further detail with reference to the accompanying drawings.

Hereinafter, examples of the present invention are described in detail with reference to the accompanying drawings so that those skilled in the art can easily carry out the present invention. The present invention may be embodied in various different forms and is not limited to the examples described herein.

In order to clearly illustrate the present invention in the drawings, parts not related to the description are omitted, and the same or similar components are denoted by the same reference numerals throughout the specification.

Throughout the specification, when a part is referred to as being "connected" to another part, it includes not only "direct connection" but also "indirect connection" where another member is placed between them. In addition, when a part is referred to as "comprising" a constituent, it does not exclude other constituents and may further include other constituents unless specifically stated to the contrary.

The terms used herein are defined in consideration of the functions of the present invention and may vary depending on the intention or custom of a user or an operator. Therefore, the definitions of these terms are meant to be interpreted in accordance with the meaning and concept consistent with the technical aspects of the present invention.

Additionally, optional terms in the following examples are used to distinguish one constituent from another, and the constituent is not limited by the terms. In the following description of the present invention, detailed descriptions of related publicly known arts which may unnecessarily obscure the gist of the present invention are omitted.

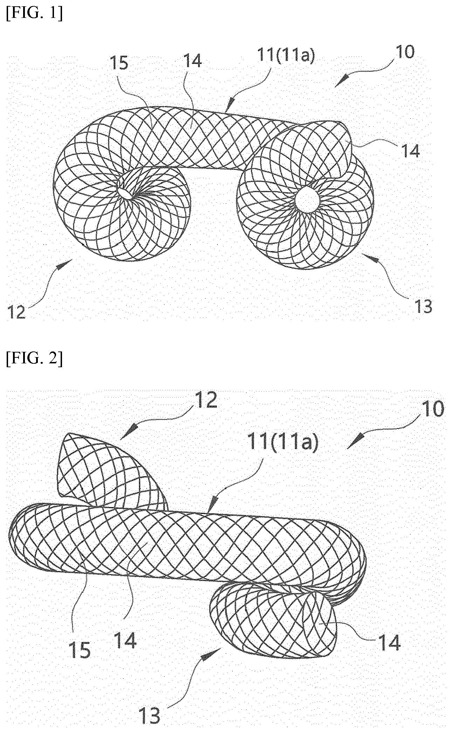

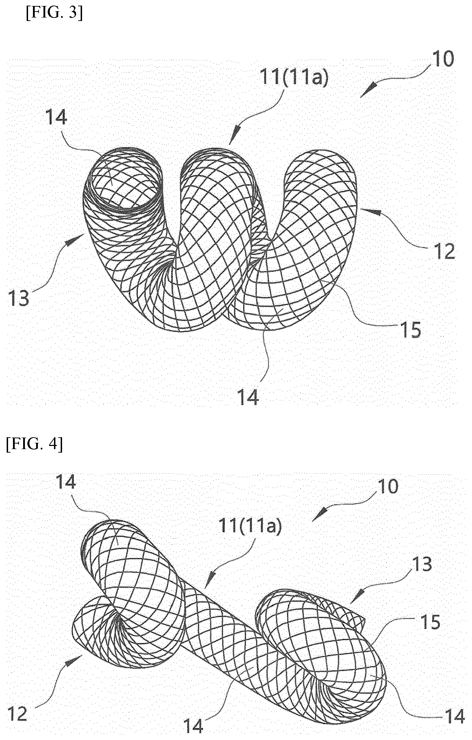

FIG. 1 is a front view of an example of the stent for connection between organs of different kinds with a pigtail structure according to the present invention. FIG. 2 is a top view of an example of the stent for connection between organs of different kinds with a pigtail structure of the present invention. FIG. 3 is a right view of an example of the stent for connection between organs of different kinds with a pigtail structure of the present invention. FIG. 4 is a bottom view of an example of the stent for connection between organs of different kinds with a pigtail structure of the present invention.

A stent (10) for connection between organs of different kinds with a pigtail structure according to the present invention is used to connect organs of different kinds. In the present example, for the organs of different kinds a particular case is described as an example in which it is used for connection between organs of different kinds where a pseudocyst (or gallbladder, liver abscess, biliary tract or the like) (20) and the duodenum (or stomach) (30) are directly connected.

The stent (10) for connection between organs of different kinds with a pigtail structure according to the present invention is made of a stent body (11) where a connecting passage (11a) with a mesh structure is formed, a one-side wound end part (12) and an other-side wound end part (13) which are formed at both end parts of the stent body to be caught and fixed after installation, and a cover film (14) made of silicon which envelops the mesh structure. The cover film (14) is made by dipping the stent body (11) to polymer solution or by overlaying a preformed polymer film on the stent body (11).

Where a through hole (21, 31) is formed on each of a pseudocyst (or gallbladder, liver abscess, biliary tract or the like) (20) and the duodenum (or stomach) (30), the stent body (11) is used for connection of portions around the through hole (21, 31) to each other, and it has a mesh structure, with a length of 20-40mm and a diameter of 6-12mm, and is provided as an integral body of woven wire (15), and both end parts are arranged across the through holes formed on the pseudocyst (or gallbladder, liver abscess, biliary tract or the like) (20) and the duodenum (or stomach) (30), and a connecting passage (11a), which connects the pseudocyst (or gallbladder, liver abscess, biliary tract or the like) (20) and the duodenum (or stomach) (30), is formed.

The one-side wound end part (12) and the other-side wound end part (13) are provided as an integral body with a mesh structure of woven wire (15) and are formed in a pigtail shape at both end parts of the stent body (11) to be caught and fixed to the through holes (21, 31), such that portions of the pseudocyst (or gallbladder, liver abscess, biliary tract or the like) (20) and the duodenum (or stomach) (30) around the through holes (21, 31) can be in close contact with each other.

The cover film has a thickness of 4-8mm and is made of silicon to cover the woven wire (15) for sealing of the connecting passage (11a).

The total length of the stent comprising the stent body (11), the one-side wound end part (12), and the other-side wound end part (13) is preferably 130-150mm, and a shape-memory alloy, a metal, or a synthetic resin material may be used for the wire (15), with its thickness suitably being 0.1-0.2mm.

It is preferable that from the stent body (11), the one-side wound end part (12) is wound 360 degrees from the top right toward the bottom right and the other-side wound end part (13) is wound 360 degrees from the bottom left toward the top left for prevention of unwinding.

However, the above does not limit the winding angles of the one-side wound end part (12) and the other-side wound end part (13), and they may obviously be used in a state where they are wound 90 to 359 degrees as necessary.

This is because, if winding angles of the one-side wound end part (12) and the other-side wound end part (13) are too small, there is a risk that the stent body (11) is released from the pseudocyst (or gallbladder, liver abscess, biliary tract or the like) (20) and the duodenum (or stomach) (30).

FIGs. 1 to 5 illustrate and describe an optimal example where from the stent body (11), the one-side wound end part (12) is wound 360 degrees from the top right toward the bottom right (11) and the other-side wound end part (13) is wound 360 degrees from the bottom left toward the top left.

Although the one-side wound end part (12) and the other-side wound end part (13) are preferably wound in the opposite directions, they may obviously be wound in the same direction.

That is, a configuration is possible where from the stent body (11), the one-side wound end part (12) is wound 360 degrees from the top right toward the bottom right and the other-side wound end part (13) is wound 360 degrees from the bottom right toward the top right.

When necessary, a configuration is possible where from the stent body (11), the one-side wound end part (12) is wound 360 degrees from the top left toward the bottom left and the other-side wound end part (13) is wound 360 degrees from the bottom left toward the top left.

Additionally, a configuration is obviously possible where from the stent body (11), the one-side wound end part (12) is wound 360 degrees from the top left toward the bottom left and the other-side wound end part (13) is wound 360 degrees from the bottom right toward the top right.

And, it is obviously possible to form only the one-side wound end part (12) on one side of the stent body (11) to use it as a non-slip stent.

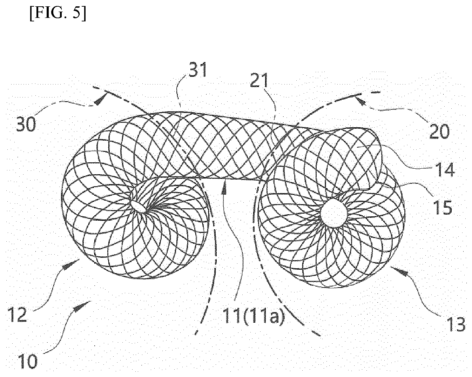

FIG. 5 is a front view of an example of the stent for connection between organs of different kinds with a pigtail structure during operation according to the present invention.

For the invention above, a through hole (21, 31) needs to be made first on each of a pseudocyst (or gallbladder, liver abscess, biliary tract or the like) (20) and the duodenum (or stomach) (30) during operation for connection of the pseudocyst (or gallbladder, liver abscess, biliary tract or the like) (20) and the duodenum (or stomach) (30).

Then, the practitioner puts a stent (10) for connection in an endoscope (not shown) and, when the front end of the endoscope reaches the through holes (21, 31) of the pseudocyst (or gallbladder, liver abscess, biliary tract or the like) (20) and the duodenum (or stomach) (30) of the subject, the practitioner pushes the stent (10) from behind into the through holes (21, 31) to transplant it, before pulling out the endoscope from the subject.

The stent (10), which was inserted and transplanted in a compressed state, stretches out in response to the body temperature or due to its own elasticity, and where both end parts of the stent (10) are placed at the through holes (21, 31) of the pseudocyst (or gallbladder, liver abscess, biliary tract or the like) (20) and the duodenum (or stomach) (30), a one-side wound end part (12) and an other-side wound end part (13) which are both end parts of the stent return to their preset shape.

Here, as shown in FIG. 5, from the stent body (11), the one-side wound end part (12) is wound 360 degrees from the top right toward the bottom right and the other-side wound end part (13) is wound 360 degrees from the bottom left toward the top left, and that state is maintained continuously.

Accordingly, the stent body (11) of the stent (10) for connection is placed between the through holes (21, 31) to form a connecting passage (11a), and they are thus arranged at the through holes (21, 31) to connect the pseudocyst (or gallbladder, liver abscess, biliary tract or the like) (20) and the duodenum (or stomach) (30), with the other-side wound end part (13) being caught and fixed to the through hole (21) of the pseudocyst (or gallbladder, liver abscess, biliary tract or the like) (20) and the one-side wound end part (12) being caught and fixed to the through hole (31) of the duodenum (or stomach) (30).

As a result, the pseudocyst (or gallbladder, liver abscess, biliary tract or the like) (20) and the duodenum (or stomach) (30) are naturally connected and joined over time without a process of incision of the abdomen of the subject or a surgery to stitch the organs.

Therefore, the subject may recover very quickly after the operation and be discharged earlier.

The examples herein and the drawings attached hereto are only to clearly describe part the technical idea that the present invention comprises, and all of modifications and specific examples that can be easily conceived by a skilled person in the art within the scope of the technical idea included in the specification of the present invention and the drawings are obviously within the scope of the technical idea of the present invention.

The scope of the present invention is defined by the following claims rather than the detailed description, and all modifications or modified forms derived from the meaning and scope of the claims and their equivalents should be construed as being included in the scope of the present invention.

### [DESCRIPTION OF REFERENCE NUMERALS]

10: stent
11: stent body
11a: connecting passage
12: one-side wound end part
13: other-side wound end part
14: cover film
15: wire
20: pseudocyst (or gallbladder, liver abscess, biliary tract or the like)
21: through hole
30: duodenum (or stomach)
31: through hole

## Claims

1. A stent with a pigtail structure used for connection between organs of different kinds where a through hole is formed on each of the organs of different kinds and portions around the through holes are connected to each other, the stent comprising:
a stent body where both end parts are arranged across the through holes formed on the organs of different kinds and a connecting passage which connects the two organs of different kinds is formed; and
a wound end part which is formed in a pigtail shape at an end part of the stent body to be caught and fixed to the through hole such that portions of the organs of different kinds around the through holes are in close contact with each other,
wherein the stent body and the wound end part are provided as an integral body having a mesh structure made of woven wire and comprising a cover film which covers the woven wire for sealing of the connecting passage.

2. The stent according to claim 1,
wherein the wound end part is a one-side wound end part formed only on one side of the end parts of the stent body.

3. The stent according to claim 1,
wherein the wound end part is a one-side wound end part and an other-side wound end part formed on both sides of the end parts of the stent body.

4. The stent according to claim 3,
wherein the one-side wound end part is wound 360 degrees from the top right toward the bottom right of the stent body and the other-side wound end part is wound 360 degrees from the bottom left toward the top left of the stent body.

5. The stent according to claim 3,
wherein the one-side wound end part is wound 360 degrees from the top right toward the bottom right of the stent body and the other-side wound end part is wound 360 degrees from the bottom right toward the top right of the stent body.

6. The stent according to claim 3,
wherein the one-side wound end part is wound 360 degrees from the top left toward the bottom left of the stent body and the other-side wound end part is wound 360 degrees from the bottom left toward the top left of the stent body.

7. The stent according to claim 3,
wherein the one-side wound end part is wound 360 degrees from the top left toward the bottom left of the stent body and the other-side wound end part is wound 360 degrees from the bottom right toward the top right of the stent body.

8. The stent according to claim 3,
wherein the stent body has a length of 20-40mm and a diameter of 6-12mm, the total length of the stent body, the one-side wound end part, and the other-side wound end part is 130-150mm, and the cover film has a thickness of 4-8mm.
